# EUROPEAN PATENT SPECIFICATION

(11) **EP 1 613 239 B1**
(45) Date of publication and mention of the grant of the patent: **30.06.2010**
(21) Application number: 04719282.8
(22) Date of filing: 10.03.2004
(51) Int. Cl.: A61F 2/01

(54) **EMBOLIC FILTER LOOP FABRICATED FROM COMPOSITE MATERIAL**
DRAHT AUS VERBUNDWERKSTOFF FÜR EMBOLISCHEN FILTER
BOUCLE DE FILTRE EMBOLIQUE FABRIQUE A PARTIR D'UNE MATIERE COMPOSITE

(30) Priority: 11.04.2003 US 410896
(43) Date of publication of application: 11.01.2006
(73) Proprietor: Boston Scientific Limited, St. Michael (BB)
(72) Inventor: BOISMIER, Dennis, A., Shorewood, Minnesota 55331 (US); PALLAZZA, Stefan, Maple Grove, Minnesota 55369 (US)
(74) Representative: Vossius & Partner
(86) International application number: PCT/US2004/007361
(87) International publication number: WO 2004/093742

(56) References cited:
- EP-A- 0 809 998
- WO-A-93/19803
- US-A- 5 800 511
- US-A1- 2002 055 747
- US-A1- 2002 188 314
- US-A1- 2003 004 536
- US-B1- 6 544 279

## Description

The present invention relates generally to the field of embolic protection devices. More specifically, the present invention relates to embolic protection filters fabricated from composite materials having certain radiopaque and elasticity characteristics.

Intravascular devices such as embolic protection filters are generally placed in a body lumen such as an artery or vein, downstream of a therapeutic site to filter emboli contained in the blood. In a typical procedure such as percutaneous transluminal coronary angioplasty (PTCA), an embolic protection filter is loaded into a delivery device such as a catheter or sheath, and advanced in a collapsed position to a location downstream of a lesion. Once positioned downstream the lesion, the embolic protection filter is ejected from the delivery device and deployed in the blood vessel. An angioplasty catheter containing an angioplasty balloon is then advanced along the guidewire and positioned across the site of the lesion to be dilated. The angioplasty balloon is then inflated, forcing the lesion to rupture and dislodge from the wall of the vessel. The dislodged debris is then carried downstream towards the embolic protection filter, where it can be collected and stored for later removal.

A number of embolic protection filters have been developed to filter embolic debris contained in the blood stream. Typically, these devices contain a support structure coupled to a filter membrane that filters contaminants in the blood stream. The support structure generally includes a number of wires, ribs, or struts formed of a relatively elastic material that can be used to support the filter membrane in an expanded position within the vessel. At the conclusion of the therapeutic procedure, the embolic protection filter is then collapsed within a retrieval catheter and removed from the vessel.

To monitor the placement and operation of the embolic protection filter, a radiopaque coil or marker band disposed on the filter can be used to produce a radiopaque image on a fluoroscopic monitor located outside of the patient's body. In one technique, for example, a radiopaque coil comprising a relatively dense metal such as platinum or tantalum is placed about the portion of the filter frame that forms the proximal mouth of the filter where embolic debris enters the filter membrane. In use, the radiopaque coil produces a bright image on the fluoroscopic monitor, allowing the operator to visualize the filter within the body. While many designs in the art have focused on the use of separate radiopaque elements to provide the necessary radiopacity, such designs often entail added manufacturing and processing steps, adding to the complexity and, in some cases, the size of the embolic protection filter. As such, it is desirable to have a filter support structure formed of composite materials exhibiting certain desirable radiopaque and elasticity characteristics.

An embolic protection filter is disclosed in US-B-6544279.

The present invention relates to embolic protection filters fabricated from composite materials according to claim 1. An embolic protection filter in accordance with an exemplary embodiment of the present invention includes a support structure and a filter membrane for filtering embolic debris contained in the blood. The support structure may comprise a wire loop formed of a composite member having one or more outer layer(s) surrounding an inner core member. The various layers may be formed from materials exhibiting certain desirable characteristics such as elasticity, radiopacity and biocompatibility. In certain embodiments, the support structure may include a bend region formed by grinding away a portion of the outer surface of the composite member. Methods according to claim 17 of forming the composite member, including drawing and cladding, are also disclosed.

### Brief Description of the Drawings

Figure 1 a perspective view of an embolic protection filter in accordance with an exemplary embodiment of the present invention;
Figure 2 is a perspective view of the wire loop of Figure 1, showing the wire loop in an expanded position;
Figure 3 is a cross-sectional view of the wire loop along line 3-3 of Figure 2;
Figure 4 is a cross-sectional view of a wire loop in accordance with the present invention, wherein the wire loop is formed of a composite member having three layers;
Figure 5 is a perspective view of a wire loop in accordance with an exemplary embodiment of the present invention, wherein the wire loop includes a bend region; and
Figure 6 is a graph of stress as a function of strain for a composite wire loop placed under tension.

### Detailed Description of the Invention

The following description should be read with reference to the drawings, in which like elements in different drawings are numbered in like fashion. The drawings, which are not necessarily to scale, depict selected embodiments and are not intended to limit the scope of the invention. Although examples of construction, dimensions, and materials are illustrated for the various elements, those skilled in the art will recognize that many of the examples provided have suitable alternatives that may be utilized.

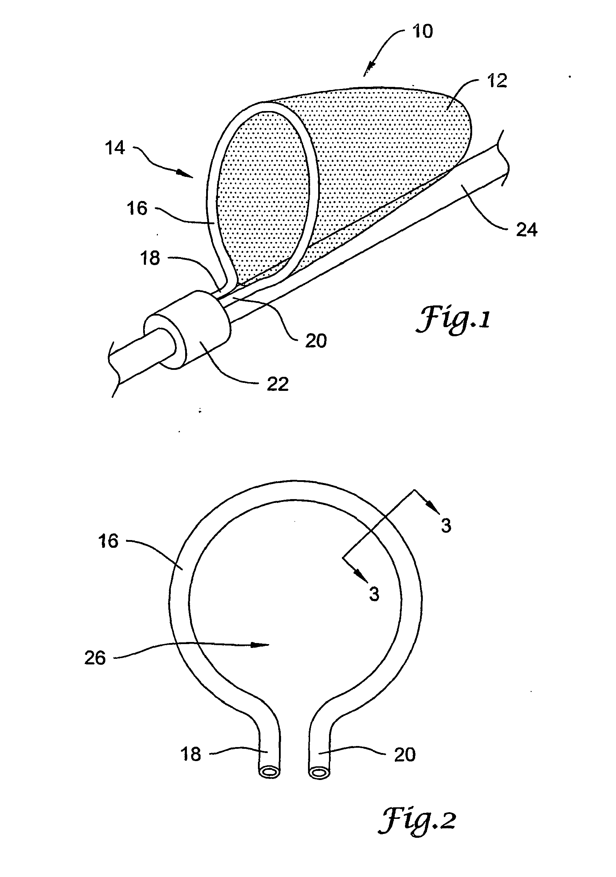

Figure 1 is a perspective view of an embolic protection filter 10 in accordance with an exemplary embodiment of the present invention. Embolic protection filter 10 comprises a filter membrane 12 coupled to a filter frame 14 or other support structure configured to support the filter membrane 12 in an expanded position within a body vessel. Filter frame 14 includes a wire loop 16 formed of a composite member having radiopaque properties that permits the wire loop 16 to be visualized via fluoroscopy. The wire loop 16 also has certain desirable elasticity characteristics that permit the embolic protection filter 10 to be radially collapsed within a delivery device such as a catheter or sheath. As shown in Figure 1, the ends 18,20 of the wire loop 16 may be attached to a tubular member 22 configured to slide along a guidewire 24 or other guiding member within the body, allowing movement of the embolic protection filter 10 relative to the guidewire 24. In an alternative embodiment (not shown), the ends 18,20 of the wire loop 16 can be fixedly secured directly to the guidewire 24, in which case the guidewire 24 can be used to advance the embolic protection filter 10 within the body.

Figure 2 is a perspective view of the wire loop 16 of Figure 1, showing the wire loop 16 in an expanded position. As illustrated in Figure 2, wire loop 16 is formed from a single, composite wire member having a circular, hoop-like shape that forms an opening 26 that receives embolic debris contained in the bloodstream. The size of the opening 26 can be selected to permit the embolic protection filter 10 to be deployed in various locations within the body, such as the coronary or peripheral arteries.

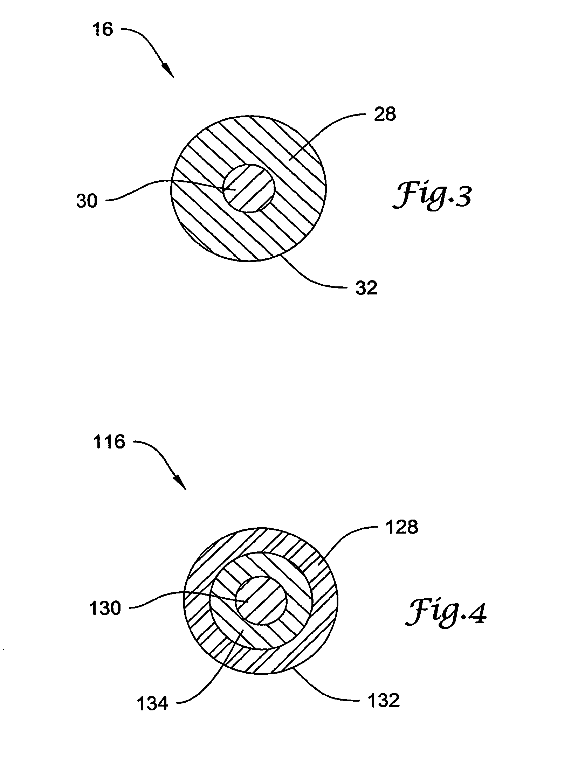

Figure 3 is a transverse cross-sectional view of the wire loop 16 along line 3-3 in Figure 2. As can be seen in Figure 3, wire loop 16 may comprise a composite wire member having an outer layer 28 concentrically disposed about an inner core member 30. The outer layer 28 and inner core member 30 may be formed of differing materials selected to exhibit various desirable mechanical properties, including stiffness (*i.e*. modulus of elasticity), biocompatibility, durability, tensile strength, compressive strength, and radiopacity. For example, the outer layer 28 may be formed of a superelastic or pseudoelastic metal such as nickel-titanium alloy (Nitinol), which exhibits characteristics such as high elasticity and biocompatibility. Superelastic metals such as Nitinol are capable of providing excellent resistance to bio-corrosion while providing a greater level of elasticity than other metals frequently used in the art, such as stainless steel or titanium. Other superelastic materials that can be used to form the outer member 28 include silver-cadmium (Ag-Cd), gold-cadmium (Au-Cd), gold-copper-zinc (Cu-Au-Zn), copper-aluminum-nickel (Cu-Zn-Al), copper-gold-zinc (Cu-Au-Zn), copper-zinc (Cu-Zn), copper-zinc-aluminum (Cu-Zn-Al), copper-zinc-tin (Cu-Zn-Sn), copper-zinc- silicon (Cu-Zn-Si), iron-beryllium (Fe₃Be); iron-nickel-titanium-cobalt (Fe-Ni-Ti-Co), iron-platinum (Fe₃Pt), indium-thallium (In-Tl), iron-manganese (Fe-Mn), nickel-titanium-cobalt (Ni-Ti-Co), and copper-tin (Cu-Sn).

While superelastic alloys have certain desirable biocompatibility and elasticity characteristics, it should be understood that the outer layer 28 may be formed of any number of suitable materials, including stainless steel (*e.g.* type 304 or 316), titanium, Beta III Titanium, or precursors of superelastic alloys. Precursor alloys are similar in chemical composition to superelastic alloys, but have not been processed to impart superelasticity during use, thereby exhibiting linear stress-strain characteristics in the body. Such alloys are further described in co-owned U.S. Patent No. 5,238,004 to Sahatjian et al.

The outer layer 28 may also be configured to exhibit certain shape-memory properties within the body. Using a superelastic material such as Nitinol, wire loop 16 can be configured to assume a particular shape, such as that illustrated in Figure 2, when deployed from the delivery catheter and exposed to temperature within the body. The shape-memory material may be binary or ternary in form, and can be configured such that the wire loop 16 reverts back to a collapsed shape when loaded into the delivery device and cooled using, for example, a cooling fluid.

The outer surface 32 of the wire loop 16 may also include a lubricious (*e.g*. hydrophilic) coating such as polytetraflouroethylene (PTFE) to permit the wire loop 16 to be easily collapsed within the delivery device, and to reduce trauma to the vessel wall during deployment. Moreover, the wire loop 16 may include an anti-thrombogenic agent such as heparin (and its derivatives), urokinase, or PPack (dextrophenylalanine proline arginine chloromethylketone) to reduce thrombosis from occurring when the wire loop 16 is placed into contact with the vessel wall.

The inner core member 30 may comprise a metal or metal/polymer blend having a density generally greater than that of the outer layer 28 to enhance the radiopacity of the wire loop 16. The inner core 30 may be formed of a continuous solid mass extending through the entire length of the wire loop 16, or may be in a powder-form. Examples of some materials that can be used as the inner core member 30 include bismuth, gold, iridium, palladium, platinum, silver, stainless steel, tantalum, titanium, tungsten, or alloys of these materials. The inner core member 30 and/or the outer layer 28 may also be loaded with a radiopaque filler material such as bismuth (Bi), barium (*e.g*. BaSO₄), or tungsten (W) to enhance the radiopacity of the wire loop 16, if desired.

In certain examples, the inner core member 30 may have a higher modulus of elasticity, and hence greater stiffness, than the material used to form the outer layer 28. By way of example, the inner core member 30 may comprise platinum having a modulus of elasticity of about 171 GPa whereas the outer member 28 may comprise a nickel-titanium alloy having a modulus of elasticity of about 40-75 GPa, depending on whether the material is in its martensite or austenite phase. Generally, the smaller the difference between the modulus of elasticity of the outer layer 28 and the inner core member 30, the smaller the variation of modulus of the overall wire support 16 in comparison to support structures containing a single (*i.e*. non-composite) material. For larger variations, the size of the inner core member 30 can be reduced to produce a wire loop 16 in which the elastic properties of the wire loop 16 are dominated by the outer layer 28.

The location of the various layers 28,30 used to form the composite wire loop 16 may also be changed to alter the particular mechanical characteristics of the device. For example, the outer layer 28 may comprise a radiographically dense material to enhance the radiopacity of the composite wire loop 16 whereas the inner core member 30 may comprise a relatively elastic material such as superelastic nickel-titanium alloy to impart elasticity to the wire loop 16. The location of each layer forming the composite may be selected to change one or more properties of the wire loop 16, such as tensile strength, compressive strength, bendability, durability or torquability.

Figure 4 is a cross-sectional view of a wire loop 116 in accordance with the present invention, wherein wire loop 116 is formed of a composite wire member having three layers 128,130,134. Similar to the example of Figures 2-3, wire loop 116 may include an outer layer 128 formed of a material having certain desirable elasticity and biocompatibility characteristics, and an inner core member 130 formed of a relatively dense material (*e.g*. platinum) to impart the desired radiopacity and resolution to the wire loop 116. Alternatively, the location of the layers may be interchanged such that the outer layer 128 is be formed of a relatively dense material whereas the inner core member 130 is formed of a material having certain desirable elasticity characteristics. In either embodiment, the outer surface 132 of the wire loop 116 may also optionally include an anti-thrombogenic agent or lubricious coating to reduce trauma to the vessel wall.

Wire loop 116 may further include a third, intermediate layer 134 disposed between the outer layer 128 and inner core member 130 selected to exhibit various properties described herein. In certain examples the intermediate layer 134 may be formed from a material having a modulus of elasticity between that of the outer layer 128 and inner core member 130 to blend the stiffness transversely across the wire loop 116. According to the invention the intermediate layer 134 is formed of a material that acts as a tie-layer to more effectively bond the outer layer 128 to the inner core member 130.

While the exemplary embodiments illustrated herein show composite wire loops having a transverse cross-sectional dimension that is substantially circular in shape, other configurations such as square, triangular, hexagonal, octagonal, trapezoidal, etc. may be employed. For example, the outer layer(s) of the composite wire loop may be rectangular in cross-sectional area with a rectangular-shaped inner core member. In some embodiments, the wire loop may be formed from composite tubing, wherein the inner layer(s) of the tubing can be formed of materials having the desired radiopacity characteristics whereas the outer layer(s) exhibit the desired elasticity properties. The wire loop may comprise a single strand of composite wire, or may comprise multiple composite members that are wound or braided together, forming a multifilament wire loop.

The wire loop can be drawn-formed, according to the invention, by removing the center portion of a rod comprised of the outer layer material to form a bore through the rod, and then inserting a smaller rod of inner core material through the bore. With the ends of the wire loop sealed (*e.g*. by crimping), the wire loop is then drawn through a series of dies of decreasing diameter until the desired dimensions of the wire loop are achieved. The drawn-formed wire loop can then be subjected to various heat-treatment steps, if desired, to anneal, harden, or impart superelastic and/or shape-memory properties to the layers. Such processes are described in co-owned U.S. Patent No. 5,628,787 to Mayer.

Other manufacturing methods such as cladding or plating are also possible, depending on the particular type of materials used and mechanical properties desired. In a cladding process, for example, one or more metallic outer layers and a metallic inner core member having certain desirable radiopacity and elasticity characteristics can be bonded together at a suitable temperature and pressure, causing the various layers to diffuse together at each interface to form an alloy.

The type of materials used to form the outer layer(s) and the inner core member of the wire loop can be selected based on the particular manufacturing process implemented. For example, a drawing process may be particularly desirable for forming composite wire loops formed of superelastic materials, such as Nitinol, imparted with shape-memory and/or superelasticity characteristics that may be affected at certain temperatures.

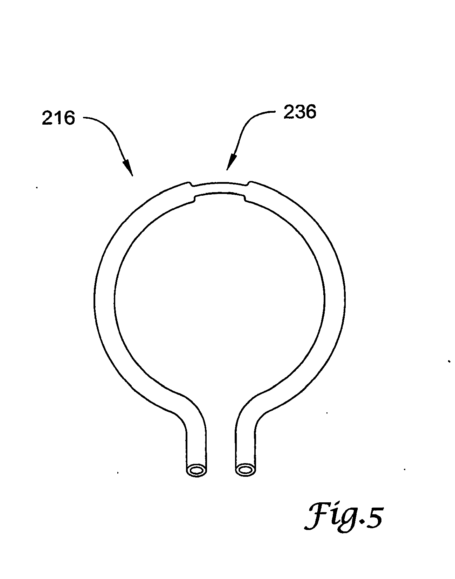

According to the invention a wire loop is first formed, for example, by a drawing or cladding process, and then subsequently ground at a middle region to form a bend region on the wire loop. As shown in Figure 5, for example, a wire loop 216 similar to that described above with respect to Figures 2-3 may include a bend region 236 formed by grinding away a portion of the outer surface of the wire loop 216. Because of the reduction in diameter of the wire loop 216 at the bend region 236, the wire loop 216 can be subjected to further bending, and in some cases, loaded into a delivery device having a smaller profile.

### EXAMPLE

An exemplary composite wire loop suitable for use in an embolic protection filter may be formed as follows. A 0,025 mm (0.0010-inch) to 0,254 mm (0.0100-inch) diameter drawn-formed composite wire may include a nickel-titanium outer layer that forms about 80% of the total transverse cross-sectional area of the wire loop, and an inner core member comprising a 90% platinum (Pt) 10% nickel (Ni) metal composition that forms the remaining 20% of the total transverse cross-sectional area of the wire loop.

Once drawn, the composite wire member is then inserted into a shaping mandrel having a shape similar to the final, formed wire loop illustrated, for example, in Figure 2. The shaping mandrel and accompanying composite wire is then subjected to a heating process wherein the composite wire is heated to a temperature of 500°C for a period of approximately 10 minutes. During this time, the nickel-titanium outer layer is heated to a temperature above the austenitic finish temperature A_{f} *(i.e.* the temperature at which the material completely transforms to austenite), thereby setting the hoop-like shape of the composite wire. Once the desired shape-memory and superelasticity characteristics have been imparted to the nickel-titanium outer layer, the composite wire member is then cut to a specific length and attached to the other filter components to form the embolic protection filter.

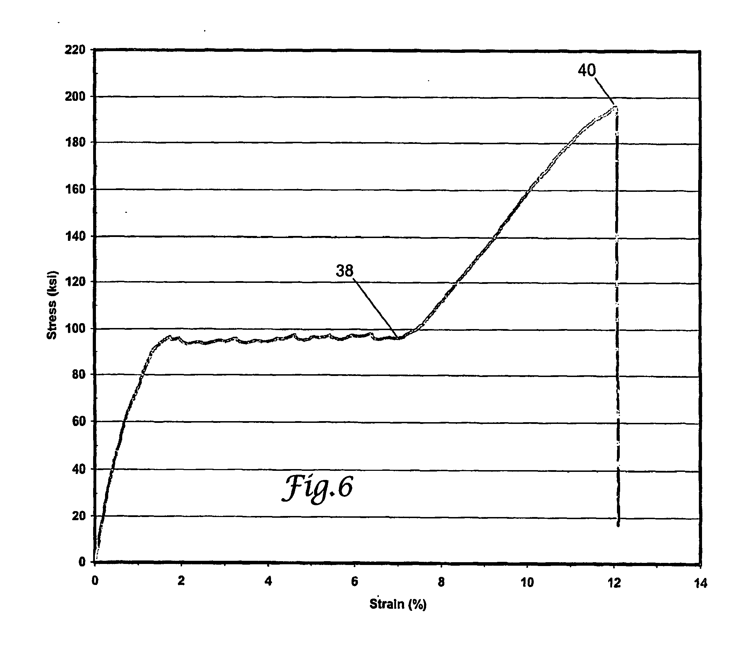

Referring now to Figure 6, a tensile stress-strain curve is shown for the composite wire loop. As shown in Figure 6 the composite wire loop reaches a relatively constant loading plateau at about 6,55 MPa (95 ksi) of applied stress, wherein the stress level of the composite wire remains relatively constant. At point 38, after about 7% strain, the composite wire loop reaches its plastic yield point, thereafter forming a permanent set in the material. At point 40, at about 1,34 GPa (195 ksi) of applied stress, the composite wire loop reaches its ultimate tensile strength, causing the wire loop to break. As illustrated in Figure 6, the desired superelasticity characteristics of the composite wire loop are maintained, as evidenced by the constant stress plateau at about 65,5 MPa (95 ksi), despite the presence of the platinum inner core member.

Having thus described the several embodiments of the present invention, those of skill in the art will readily appreciate that other embodiments may be made and used which fall within the scope of the claims attached hereto. Numerous advantages of the invention covered by this document have been set forth in the foregoing description. It will be understood that this disclosure is, in many respects, only illustrative. Changes may be made in details, particularly in matters of shape, size and arrangement of parts without exceeding the scope of the invention.

## Claims

1. An embolic protection filter (10) for collecting embolic debris within a blood vessel, said embolic protection filter (10) comprising:
a guidewire (24);
a tubular member (22) configured to slide along the guidewire (24);
a support structure forming a proximal month of the embolic protection filter, said support structure including a wire loop (16) formed of a composite member having at least a first layer (128) formed of a first material, and an inner core member (130) disposed within the first layer formed of a second material different than the first material, the second material having a radiographic density greater than the first material to enhance the visibility of the embolic protection filter (10); wherein the wire loop (16) is attached to the tubular member; and
a filter membrane (12) operatively coupled to the support structure ; **characterized in that** said composite member further comprises an intermediate layer (134) between the first layer and the inner core member, the intermediate layer formed of a material that acts as a tie layer to more effectively bond the outer layer to the inner core member.

2. The embolic protection filter of claim 1, wherein said composite member is formed at least in part of a shape-memory material configured to deform to a pre-set shape when deployed in the body.

3. The embolic protection filter of claim 1, wherein said first material is a superelastic material.

4. The embolic protection filter of claim 3, wherein said superelastic material is nickel-titanium alloy.

5. The embolic protection filter of claim 3, wherein said superelastic material is selected from the group consisting of silver-cadmium, gold-cadmium, gold-copper-zinc, copper-aluminum-nickel, copper-gold-zinc, copper-zinc, copper-zinc-aluminum, copper-zinc-tin, copper-zinc-silicon, iron-beryllium, iron-nickel-titanium-cobalt, iron-platinum, indium-thallium, iron-manganese, nickel-titanium-cobalt, and copper-tin.

6. The embolic protection filter of claim 1, wherein said second material is platinum.

7. The embolic protection filter of claim 1, wherein said second material is selected from the group consisting of bismuth, gold, iridium, platinum, rhenium, silver, tantalum, and tungsten.

8. The embolic protection filter of claim 1, wherein said composite member includes a bend region.

9. The embolic protection filter of claim 1, wherein said composite member includes a lubricious coating.

10. The embolic protection filter of claim 1, wherein said composite member includes an anti-thrombogenic coating.

11. The embolic protection filter of claim 1, wherein the transverse cross-sectional dimension of said composite member is about 25.4 µm to 254 µm (0.0010 inches to 0.0100 inches).

12. The embolic protection filter of claim 1, wherein said composite member is configured to expand to a circular shape when deployed within the blood vessel.

13. The embolic protection filter of claim 1, wherein said second material can be visualized within the blood vessel using fluoroscopy.

14. The embolic protection filter of claim 1, wherein the first material is formed of a superelastic alloy having shape-memory characteristics at body temperature.

15. The embolic protection filter of claim 1, wherein the support structure comprises a composite wire loop (16).

16. The embolic protection filter of claim 15, wherein the first material is formed of radiopaque material configured to enhance the visibility of the embolic protection filter, and the second material comprises a superelastic alloy having shape-memory characteristics at body temperature.

17. A method for making an embolic filter according to any of claims 1 to 16, the method comprising:
providing a guidewire with a tubular member configured to slide along the guidewire;
forming a support structure by:
removing a center portion of a rod comprised of a first material;
inserting a core rod comprising of a second material with a greater radiographic density into said first material;
drawing or cladding the first material to the second material;
grinding to form a bend region on said embolic filter loop; and
attaching the support structure to the tubular member.

## Patentansprüche

1. Embolieprotektionsfilter (10) zum Auffangen von Embolietrümmern in einem Blutgefäß, wobei das Embolieprotektionsfilter (10) aufweist:
einen Führungsdraht (24);
ein Röhrenteil (22), das so konfiguriert ist, daß es entlang dem Führungsdraht (24) gleitet;
einen Stützaufbau, der eine proximale Mündung des Embolieprotektionsfilters bildet, wobei der Stützaufbau eine Drahtschlaufe (16) aufweist, die gebildet ist aus einem Verbundteil mit mindestens einer ersten Schicht (128), die aus einem ersten Material gebildet ist, und einem in der ersten Schicht angeordneten Innenkernteil (130), das aus einem zweiten Material gebildet ist, das sich vom ersten Material unterscheidet, wobei das zweite Material eine größere Röntgendichte als das erste Material hat, um die Sichtbarkeit des Embolieprotektionsfilters (10) zu verstärken; wobei die Drahtschlaufe (16) am Röhrenteil angebracht ist; und
eine Filtermembran (12), die mit dem Stützaufbau betrieblich gekoppelt ist;
**dadurch gekennzeichnet, daß** das Verbundteil ferner eine Zwischenschicht (134) zwischen der ersten Schicht und dem Innenkernteil aufweist, wobei die Zwischenschicht aus einem Material gebildet ist, das als Verbindungsschicht wirkt, um die Außenschicht mit dem Innenkernteil wirksamer zu verbinden.

2. Embolieprotektionsfilter nach Anspruch 1, wobei das Verbundteil mindestens teilweise aus einem Formgedächtnismaterial gebildet ist, das so konfiguriert ist, daß es sich bei Einsatz im Körper in eine voreingestellte Form verformt.

3. Embolieprotektionsfilter nach Anspruch 1, wobei das erste Material ein superelastisches Material ist.

4. Embolieprotektionsfilter nach Anspruch 3, wobei das superelastische Material Nickel-Titan-Legierung ist.

5. Embolieprotektionsfilter nach Anspruch 3, wobei das superelastische Material aus der Gruppe ausgewählt ist, die aus Silber-Cadmium, Gold-Cadmium, Gold-Kupfer-Zink, Kupfer-Aluminium-Nickel, Kupfer-Gold-Zink, Kupfer-Zink, Kupfer-Zink-Aluminium, Kupfer-Zink-Zinn, Kupfer-Zink-Silicium, Eisen-Beryllium, Eisen-Nickel-Titan-Cobalt, Eisen-Platin, Indium-Thallium, Eisen-Mangan, Nickel-Titan-Cobalt und Kupfer-Zinn besteht.

6. Embolieprotektionsfilter nach Anspruch 1, wobei das zweite Material Platin ist.

7. Embolieprotektionsfilter nach Anspruch 1, wobei das zweite Material aus der Gruppe ausgewählt ist, die aus Bismut, Gold, Iridium, Platin, Rhenium, Silber, Tantal und Wolfram besteht.

8. Embolieprotektionsfilter nach Anspruch 1, wobei das Verbundteil einen Biegungsbereich aufweist.

9. Embolieprotektionsfilter nach Anspruch 1, wobei das Verbundteil eine gleitfähige Beschichtung aufweist.

10. Embolieprotektionsfilter nach Anspruch 1, wobei das Verbundteil eine antithrombogene Beschichtung aufweist.

11. Embolieprotektionsfilter nach Anspruch 1, wobei das Querschnittmaß des Verbundteils etwa 25,4 µm bis 254 µm (0,0010 Inch bis 0,0100 Inch) beträgt.

12. Embolieprotektionsfilter nach Anspruch 1, wobei das Verbundteil so konfiguriert ist, daß es bei Einsatz im Blutgefäß in eine Kreisform expandiert.

13. Embolieprotektionsfilter nach Anspruch 1, wobei das zweite Material mit Hilfe von Fluoroskopie im Blutgefäß visualisiert werden kann.

14. Embolieprotektionsfilter nach Anspruch 1, wobei das erste Material aus einer superelastischen Legierung mit Formgedächtniskennwerten bei Körpertemperatur gebildet ist.

15. Embolieprotektionsfilter nach Anspruch 1, wobei der Stützaufbau eine Verbunddrahtschlaufe (16) aufweist.

16. Embolieprotektionsfilter nach Anspruch 15, wobei das erste Material aus röntgendichtem Material gebildet ist, das so konfiguriert ist, daß es die Sichtbarkeit des Embolieprotektionsfilters verstärkt, und das zweite Material eine superelastische Legierung mit Formgedächtniskennwerten bei Körpertemperatur aufweist.

17. Verfahren zur Herstellung eines Emboliefilters nach einem der Ansprüche 1 bis 16, wobei das Verfahren aufweist:
Bereitstellen eines Führungsdrahts mit einem Röhrenteil, das so konfiguriert ist, daß es entlang dem Führungsdraht gleitet;
Bilden eines Stützaufbaus durch:
Entfernen eines Mittelabschnitts eines Stabs, der ein erstes Material aufweist;
Einführen eines Kernstabs, der ein zweites Material mit einer größeren Röntgendichte aufweist, in das erste Material;
Ziehen oder Ummanteln des ersten Materials auf das zweite Material;
Abschleifen, um einen Biegungsbereich an der Emboliefilterschlaufe zu bilden; und
Anbringen des Stützaufbaus am Röhrenteil.

## Revendications

1. Filtre de protection embolique (10) pour collecter des débris emboliques à l'intérieur d'un vaisseau sanguin, ledit filtre de protection embolique (10) comprenant :
un fil guide (24) ;
un élément tubulaire (22) configuré pour coulisser le long du fil guide (24) ;
une structure de support formant une embouchure proximale du filtre de protection embolique, ladite structure de support comprenant une boucle de fil (16) formée avec un élément composite ayant au moins une première couche (128) formée avec un premier matériau, et un élément central interne (130) disposé à l'intérieur de la première couche formée avec un second matériau différent du premier matériau, le second matériau ayant une densité radiographique supérieure au premier matériau afin d'améliorer la visibilité du filtre de protection embolique (10) ; dans lequel la boucle de fil (16) est fixée sur l'élément tubulaire ; et
une membrane de filtre (12) couplée de manière opérationnelle à la structure de support ;
**caractérisé en ce que** ledit élément composite comprend en outre une couche intermédiaire (134) entre la première couche et l'élément central interne, la couche intermédiaire étant formée avec un matériau qui agit comme une couche de liaison pour relier plus efficacement la couche externe à l'élément central interne.

2. Filtre de protection embolique selon la revendication 1, dans lequel ledit élément composite est formé au moins en partie avec un matériau à mémoire de forme configuré pour se déformer selon une forme préétablie lorsqu'il est déployé dans le corps.

3. Filtre de protection embolique selon la revendication 1, dans lequel ledit premier matériau est un matériau super élastique.

4. Filtre de protection embolique selon la revendication 3, dans lequel ledit matériau super élastique est un alliage de nickel titane.

5. Filtre de protection embolique selon la revendication 3, dans lequel ledit matériau super élastique est choisi dans le groupe comprenant l'argent - cadmium, l'or - cadmium, l'or - cuivre - zinc, le cuivre - aluminium - nickel, le cuivre - or - zinc, le cuivre - zinc, le cuivre - zinc - aluminium, le cuivre - zinc - étain, le cuivre - zinc - silicium, le fer - béryllium, le fer - nickel - titane - cobalt, le fer - platine, l'indium - thallium, le fer - manganèse, le nickel - titane - cobalt, et le cuivre - étain.

6. Filtre de protection embolique selon la revendication 1, dans lequel ledit second matériau est du platine.

7. Filtre de protection embolique selon la revendication 1, dans lequel ledit second matériau est choisi dans le groupe comprenant le bismuth, l'or, l'iridium, le platine, le rhénium, l'argent, le tantale et le tungstène.

8. Filtre de protection embolique selon la revendication 1, dans lequel ledit élément composite comprend une région courbée.

9. Filtre de protection embolique selon la revendication 1, dans lequel ledit élément composite comprend un revêtement lubrifié.

10. Filtre de protection embolique selon la revendication 1, dans lequel ledit élément composite comprend un revêtement anti-thrombogène.

11. Filtre de protection embolique selon la revendication 1, dans lequel la dimension transversale dudit élément composite est de l'ordre d'environ 25,4 µm à 254 µm (0,0010 pouce à 0,0100 pouce).

12. Filtre de protection embolique selon la revendication 1, dans lequel ledit élément composite est configuré pour s'expanser selon une forme circulaire lorsqu'il est déployé à l'intérieur du vaisseau sanguin.

13. Filtre de protection embolique selon la revendication 1, dans lequel ledit second matériau peut être visualisé à l'intérieur du vaisseau sanguin à l'aide de la fluoroscopie.

14. Filtre de protection embolique selon la revendication 1, dans lequel le premier matériau est formé avec un alliage super élastique ayant des caractéristiques de mémoire de forme à la température corporelle.

15. Filtre de protection embolique selon la revendication 1, dans lequel la structure de support comprend une boucle de fil composite (16).

16. Filtre de protection embolique selon la revendication 15, dans lequel le premier matériau est formé avec un matériau radio-opaque configuré pour améliorer la visibilité du filtre de protection embolique, et le second matériau comprend un alliage super élastique ayant des caractéristiques de mémoire de forme à la température corporelle.

17. Procédé pour fabriquer un filtre embolique selon l'une quelconque des revendications 1 à 16, le procédé comprenant les étapes supplémentaires consistant à :
prévoir un fil guide avec un élément tubulaire configuré pour coulisser le long du fil guide ;
former une structure de support selon les étapes consistant à :
retirer une partie centrale d'une tige composée avec un premier matériau ;
insérer une tige centrale comprenant un second matériau avec une plus grande densité radiographique dans ledit premier matériau ;
emboutir ou plaquer le premier matériau sur le second matériau ;
meuler pour former une région courbée sur ladite boucle de filtre embolique ; et
fixer la structure de support sur l'élément tubulaire.
